(19)

Europäisches
Patentamt

European
Patent Office

Office européen
des brevets

(11)  **EP 4 645 320 A1**

(12)  **EUROPEAN PATENT APPLICATION**
published in accordance with Art. 153(4) EPC

(43) Date of publication:
05.11.2025  Bulletin 2025/45

(21) Application number: 23909433.7

(22) Date of filing: 08.10.2023

(51) International Patent Classification (IPC):
*G16B 40/00* (2019.01)

(52) Cooperative Patent Classification (CPC):
G06F 18/15; G06F 18/2135; G16B 20/20;
G16B 20/30; G16B 40/00

(86) International application number:
PCT/CN2023/123292

(87) International publication number:
WO 2024/139499 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority:  30.12.2022  CN 202211727322

(71) Applicants:
• BGI Genomics Co., Limited
Guangdong 518083 (CN)
• BGI Biotechnology (Wuhan) Co., Ltd.
Wuhan, Hubei 430070 (CN)

(72) Inventors:
• PENG, Jiguang
Shenzhen, Guangdong 518083 (CN)
• LI, Jingrou
Shenzhen, Guangdong 518083 (CN)
• XIANG, Jiale
Shenzhen, Guangdong 518083 (CN)
• SHEN, Jiankun
Shenzhen, Guangdong 518083 (CN)
• SUN, Jun
Shenzhen, Guangdong 518083 (CN)
• PENG, Zhiyu
Shenzhen, Guangdong 518083 (CN)

(74) Representative: Inspicos P/S
Agern Allé 24
2970 Hørsholm (DK)

(54)  **METHOD FOR DETECTING CHROMOSOME COPY-NUMBER VARIANT**

(57)  The present invention provides a method for detecting a chromosome copy-number variant (CNV). The method comprises: performing PCA noise reduction and CNV analysis on sequencing data of a sample to be detected, so as to detect whether said sample contains a CNV and/or determine a CNV source, wherein the PCA noise reduction is performed by comparing the sequencing data of said sample with a reference data set, and the reference data set is a principal component feature which represents noise and is obtained after PCA learning is performed on a predetermined sample. According to the method, a CNV can be accurately detected, and the occurrence source of the CNV can be determined, so that false positive or false negative results caused by a maternal CNV in a sample to be detected are effectively avoided, and false detection or missing detection of congenital defects of fetuses is prevented.

Figure 1

EP 4 645 320 A1

**Description**

**Technical Field**

The present invention relates to the field of bioinformatics, and specifically, to a method, system, electronic device and computer-readable storage medium for detecting a chromosome copy-number variant of a sample to be tested.

**Background Art**

Copy-number variant (CNV) is a structural variant in which a chromosome DNA fragment is missing or repeated, and is widely distributed in the human genome. During pregnancy, the cell-free DNA contained in the mother's peripheral blood contains fragments from the fetus. When the fetus or the mother carries CNV, the proportion of DNA fragments of the chromosome segment corresponding to the CNV in the cell-free DNA will increase or decrease accordingly. There are many common diseases caused by CNV, such as Angelman syndrome, caused by the q11-q13 deletion of Chromosome 15; cri-du-chat syndrome, caused by the short-arm deletion of Chromosome 5; DiGeorge syndrome, caused by the q11.2 deletion of Chromosome 22. The occurrence of these diseases brings great pain to the patients themselves and their families. Because the risk of diseases caused by fetal CNV increases with the increase of maternal reproductive age, it is very necessary to perform CNV detection on older pregnant women. At present, with the advancement of technology, the negative impact of traditional biopsy on the mother and fetus has been clearly recognized, and non-invasive DNA detection of CNV has been established. This technology detects fetal CNV by detecting cell-free DNA from the fetus in the plasma of pregnant women. In some cases, for example, when the sample to be tested carries large abnormal fragments such as abnormal maternal chromosome number, the false detection of some other CNVs may occur. At the same time, the current technology for determining the source of CNV only considers two scenarios: de novo in the fetus or maternal inheritance. However, when the mother carries CNV, there is a 1/2 probability of it being inherited by the fetus, and because of the possible existence of mosaicism and incomplete penetrance, even if the mother has a normal phenotype, the fetus may be negatively affected by the inherited CNV. The existing technology is not accurate enough in determining the source of CNV, and thus applying the same treatment strategy to all maternal CNVs may lead to false positives or false negatives.

Therefore, it is urgent to develop a more stable and accurate method for detecting chromosome copy-number variants that can distinguish the source of CNV in a mixed-source DNA sample, so as to accurately detect the CNV and determine the source of CNV, and avoid irreversible damage caused by false positives or false negatives.

Currently, in a patent of Annoroad company, sequencing fragments of cell-free DNA are posted back to the genome, the sequencing depth of each window is counted, the z value and CNV probability of each window are calculated, and the CNV concentration of the sample is estimated through the CNV probability, and compared with the actual fetal concentration of the sample, so as to determine whether the sample has maternal CNV. In a patent of Verinata Health, cell-free DNA fragments of different lengths are used to calculate multiple t values and likelihood ratios, detect a CNV and determine whether the CNV comes from the fetus. However, these technologies do not bring about a good method to determine the fetus's carrying status when the mother of the sample to be tested carries CNV.

**Contents of the present invention**

This application is proposed by the inventors based on the discovery of the following problems and facts: The hidden Markov model is a statistical model that can be applied to CNV detection, and XHMM is a common exome CNV analysis tool based on the hidden Markov model, which outputs potential CNV breakpoint information and has good performance in predicting CNV through sequencing data. The inventors found that this method can also be applied to cell-free DNA sequencing data of non-invasive prenatal DNA testing (NIPT).

The present invention aims to solve at least one of the technical problems in the related art to a certain extent.

The invention team of the present application breaks through the limitations of traditional detection methods and develops a set of technical methods for detecting CNV for cell-free DNA samples in pregnant women's blood. This technology plays an important role in solving problems such as chromosomal disease screening.

In the first aspect of the present invention, the present invention proposes a method for detecting chromosome copy-number variant. According to an embodiment of the present invention, the method comprises:

the sequencing data of the sample to be tested is subjected to PCA (principal component analysis) denoising processing and CNV analysis processing to detect whether the sample contains CNV and/or determine the source of CNV;

wherein, the PCA denoising processing is performed by comparing the sequencing data of the sample to be tested with a reference data set, and the reference data set is the principal component features representing the noises obtained after PCA learning of the predetermined sample. Previously, the commonly used denoising method uses the sample to be tested for PCA, and then the method of removing the first ten-dimensional principal components of PCA is used to denoise the sample. However, since PCA is an unsupervised learning method, it cannot be guaranteed that all the first ten-dimensional principal components learned are experimental or analytical noises. When the sample to be tested has a more serious abnormality, such as an abnormal number of maternal chromosomes, the PCA method will mistakenly learn the abnormal information of these windows, thereby introducing some errors when removing the first ten-dimensional principal components, thus causing abnormal CNV detection in some other intervals. The inventors found that if the PCA denoising step uses a fixed reference set sample to perform PCA learning on the principal component features representing the noise, and uses this fixed PCA result to perform denoising processing on the sample to be tested, the false detection of some other CNVs can be reduced by this method when encountering large fragment abnormalities in the sample to be tested.

According to an embodiment of the present invention, the above-mentioned method for detecting chromosome copy-number variant may also comprises at least one of the following additional technical features:

According to an embodiment of the present invention, the sample to be tested and the predetermined sample are both nucleic acid samples.

According to an embodiment of the present invention, the nucleic acid sample comprises at least one selected from DNA and RNA.

According to an embodiment of the present invention, the nucleic acid sample is derived from peripheral blood of pregnant women.

According to an embodiment of the present invention, the nucleic acid sample is cell-free DNA in peripheral blood plasma of pregnant women.

According to an embodiment of the present invention, the predetermined sample is a sample known to have no CNV.

According to an embodiment of the present invention, the sequencing data of the sample to be tested and the reference data set of the predetermined sample are obtained under identical conditions or with the same data volume. This can ensure the effectiveness of PCA learning.

According to an embodiment of the present invention, the CNV analysis processing comprises: performing CNV prediction processing on the sequencing data after PCA denoising processing; performing filtration processing on the CNV prediction processing result; performing copy-number ratio theoretical value and threshold analysis on the CNV prediction processing result after the filtration processing; and determining the source of the CNV based on the copy-number ratio theoretical value and threshold analysis result.

According to an embodiment of the present invention, the CNV prediction processing is performed using a hidden Markov model.

According to an embodiment of the present invention, the CNV prediction processing results comprise the coordinate, posterior probability, and z value information of the CNV.

According to an embodiment of the present invention, the filtration processing comprises: removing short fragments and low-reliability CNV prediction processing result based on the coordinate, posterior probability, and z value information of the CNV. The coordinate information of the CNV usually comprises the coordinate information of the starting and ending positions. By setting a threshold of the minimum CNV length, too short CNV can be filtered out. In CNV prediction, each CNV is usually accompanied by a posterior probability, indicating the credibility of the CNV prediction. A threshold of the posterior probability is set to retain CNV with high credibility. The z value indicates the degree of deviation of a CNV. A larger z value usually indicates that the CNV is significant in the sample. A threshold of the z value is set to retain significant CNV. Then the source of the CNV is accurately determined.

According to an embodiment of the present invention, the chromosome copy-number variant comprises DNA fragment deletion and DNA fragment duplication.

According to an embodiment of the present invention, when the sample to be tested is a sample with maternal CNV determined by the above-mentioned method for detecting chromosome copy-number variant, the theoretical value of the copy ratio is calculated by the following formula:

$$\text{Deletion} - \text{type copy ratio theoretical model} = \begin{cases} a + b * f & \text{maternal CNV} \\ c & \text{maternal} - \text{fetal CNV} \end{cases}$$

$$\text{Duplication} - \text{type copy ratio theoretical model} = \begin{cases} d + e * f & \text{maternal CNV} \\ j & \text{maternal} - \text{fetal CNV} \end{cases}$$

wherein, *f* is the concentration of fetal cell-free DNA in a sample with maternal source CNV, and a, b, c, d, e, j are obtained by fitting the copy-number ratio of nucleic acid sample sequencing data with maternal CNV and the concentration of fetal cell-free DNA; wherein, b, e are the slopes of the fitting curves, a, d are the intercepts of the fitting curves, and c, j are constants;

wherein, copy-number ratio = relative depth of CNV segment of sequencing data of the predetermined sample / mean relative depth of the same segment of sequencing data of the predetermined sample.

It should be noted that the maternal source described in this application is a classification including two categories of maternal and fetal. The maternal source CNV refers to that the mother (maternal body) contains CNV, and the fetal CNV content is unknown;

The maternal CNV described in this application refers to that only the mother (maternal body) contains CNV; the maternal-fetal CNV described in this application refers to that both the mother (maternal body) and the fetus contain CNV.

According to an embodiment of the present invention, the relative depth is calculated by the following formula:

Relative depth = mean read counts in the window within the CNV segment/mean read counts in all windows of the predetermined sample.

According to an embodiment of the present invention, the copy-number ratio threshold is calculated by the following formula:

Copy-number ratio threshold of deletion-type CNV = (theoretical value of copy-number ratio of maternal-fetal deletion-type CNV + theoretical value of copy-number ratio of maternal deletion- type CNV)/2;

Copy-number ratio threshold of duplication-type CNV = (theoretical value of copy-number ratio of maternal-fetal duplication-type CNV + theoretical value of copy-number ratio of maternal duplication-type CNV)/2.

According to an embodiment of the present invention, the determination of the source of CNV based on the copy-number ratio theoretical value and threshold analysis results comprises:

(1) in a deletion-type CNV, when the theoretical value of the copy-number ratio is higher than the threshold, it is an indication that the CNV comes from the mother;

(2) in a deletion-type CNV, when the theoretical value of the copy-number ratio is lower than the threshold, it is an indication that the CNV comes from the mother and the fetus;

(3) in a duplication-type CNV, when the theoretical value of the copy-number ratio is higher than the threshold, it is an indication that the CNV comes from the mother and the fetus;

(4) in a duplication-type CNV, when the theoretical value of the copy-number ratio is lower than the threshold, it is an indication that the CNV comes from the mother.

According to an embodiment of the present invention, the sequencing data of the sample to be tested is obtained in the following manner: constructing a sequencing library based on the sample to be tested; performing sequencing processing on the sequencing library to obtain the sequencing data of the sample to be tested.

According to an embodiment of the present invention, the sequencing processing is performed on the MGISEQ-2000 sequencer or BGISEQ-500 sequencer manufactured by MGI.

According to an embodiment of the present invention, the construction of the sequencing library comprises performing end repair processing, adapter ligation processing and PCR amplification processing on the sample to be tested.

According to an embodiment of the present invention, the end repair processing is performed under the condition of the presence of an end repair enzyme.

According to an embodiment of the present invention, the ligation processing comprises ligating the sample to be tested that has undergone end repair processing with a label adapter in the presence of a ligase.

According to an embodiment of the present invention, the PCR amplification processing comprises performing a PCR reaction on the sample to be tested ligated with the label adapter.

According to an embodiment of the present invention, the PCR reaction conditions comprises: 98°C, 2 minutes → (98°C, 15 seconds → 56°C, 15 seconds → 72°C, 30 seconds) 17 cycles → 72°C, 5 minutes → 4°C.

In the second aspect of the present invention, the present invention provides a system for detecting chromosome copy-number variant. According to an embodiment of the present invention, the system comprises: a PCA denoising device, which is used to perform PCA denoising processing on the sequencing data of the sample to be tested; a CNV analysis device, which is connected to the PCA denoising device, and is used to perform CNV analysis on the sequencing data after the PCA denoising processing, so as to detect CNV and/or determine the source of CNV; wherein, the PCA denoising processing is performed by comparing the sequencing data of the sample to be tested with a reference data set, and the reference data set is principal component features representing noise obtained after PCA learning of the predetermined sample. The purpose of this is to remove some errors contained in the first ten-dimensional principal components. The system according to the embodiment of the present invention is suitable for running the method for detecting chromosome copy-number variant as described in the first aspect, so as to determine the source of copy-number variant in the sample to be tested.

According to an embodiment of the present invention, the CNV analysis device comprises: a CNV prediction processing unit, which is used to perform CNV prediction processing on the sequencing data after PCA denoising processing; a filtration unit, which is connected to the CNV prediction processing unit, and is used to filter the CNV prediction processing result; a copy-number ratio analysis unit, which is connected to the filtration unit, and is used to perform copy-number ratio theoretical value and threshold analysis on the CNV prediction processing result after the filtration processing; a judgment unit, which is connected to the copy-number ratio analysis unit, and is used to determine the CNV source based on the copy-number ratio theoretical value and threshold analysis result.

According to an embodiment of the present invention, the system further comprises a sequencing library construction device and a sequencing device, in which the sequencing library construction device is used to construct a sequencing library based on the sample to be tested; the sequencing device is connected to the sequencing library construction device, and the sequencing device is connected to the PCA denoising device, and is used to sequence the sequencing library to obtain the sequencing data of the sample to be tested.

In the third aspect of the present invention, the present invention provides an electronic device for detecting chromosome copy-number variant. According to an embodiment of the present invention, the electronic device comprises: a memory, a processor, in which the processor runs a program corresponding to an executable program code by reading the executable program code stored in the memory, so as to implement the method for detecting chromosome copy-number variant as described in the first aspect of the present invention.

In the fourth aspect of the present invention, the present invention provides a computer-readable storage medium. According to an embodiment of the present invention, the computer-readable storage medium stores a computer program, and when the program is executed by the processor, the method for detecting chromosome copy-number variant as described in the first aspect of the present invention is implemented.

**Brief Description of the Drawings**

The above and/or additional aspects and advantages of the present invention will become obvious and easy to understand from the description of the embodiments in combination with the following drawings, wherein:

Figure 1 shows a system structure diagram for detecting chromosome copy-number variant according to an example of the present invention;

Figure 2 shows a structure diagram of a CNV analysis device according to an example of the present invention;

Figure 3 shows a system structure diagram for detecting chromosome copy-number variant according to another specific example of the present invention;

Figure 4 shows a CNV reporting result diagram before the improvement of the PCA denoising step in Example 1 of the present invention;

Figure 5 shows a CNV reporting result diagram after the improvement of the PCA denoising step in Example 1 of the present invention;

Figure 6 shows a relationship diagram between maternal and maternal source CNV copy ratio and fetal concentration in Example 2 of the present invention.

## Specific Models for Carrying Out the present invention

The examples of the present invention are described in details as follows, and are exemplified in the accompanying drawings. The examples described below with reference to the accompanying drawings are exemplary and are intended to be used to explain the present invention, and should not be understood as limiting the present invention.

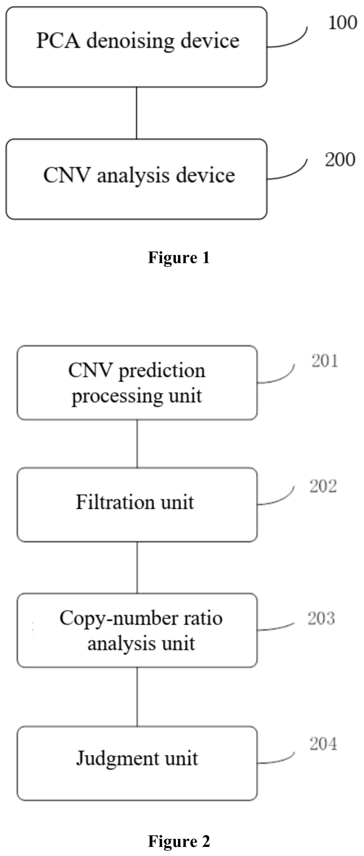

For ease of explanation, the applicant describes a system for detecting chromosome copy-number variant proposed by the present invention with reference to the accompanying drawings. According to an embodiment of the present invention, referring to Figure 1, the system comprised: a PCA denoising device 100, in which the PCA denoising device 100 was used to perform PCA denoising on the sequencing data of the sample to be tested; a CNV analysis device 200, in which the CNV analysis device 200 was connected to the PCA denoising device 100, and used to perform CNV analysis on the sequencing data after PCA denoising, so as to determine the source of CNV; wherein, the PCA denoising was performed by comparing the sequencing data of the sample to be tested with a reference data set, and the reference data set was the principal component features representing the noises obtained after PCA learning of the predetermined sample. The purpose of this was to remove some errors contained in the first ten-dimensional principal components. The system according to the example of the present invention was suitable for running the method for detecting chromosome copy-number variant as described in the first aspect to determine the source of copy-number variant in the sample to be tested.

According to the example of the present invention, referring to Figure 2, the CNV analysis device 200 comprised: a CNV prediction processing unit 201, in which the CNV prediction processing unit 201 was used to perform CNV prediction processing on the sequencing data after PCA denoising processing; a filtration unit 202, in which the filtration unit 202 was connected to the CNV prediction processing unit 201 and used to perform filtration on the CNV prediction processing result; a copy-number ratio analysis unit 203, in which the copy-number ratio analysis unit 203 was connected to the filtration unit 202 and used to perform copy-number ratio theoretical value and threshold analysis on the CNV prediction processing result after filtration processing; a judgment unit 204, in which the judgement was connected to the copy-number ratio analysis unit 203 and used to determine the CNV source based on the copy-number ratio theoretical value and threshold analysis result.

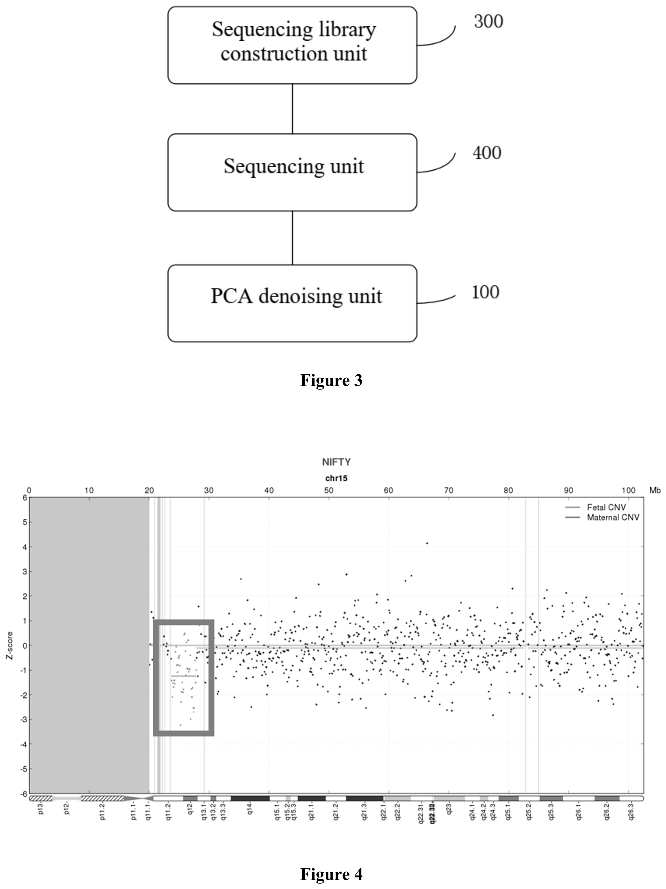

According to an example of the present invention, referring to Figure 3, the system further comprised a sequencing library construction device 300 and a sequencing device 400, wherein the sequencing library construction device 300 was used to construct a sequencing library based on the sample to be tested; the sequencing device 400 was connected to the sequencing library construction device 300, and the sequencing device 400 was connected to the PCA denoising device 100, and used to perform sequencing processing on the sequencing library so as to obtain sequencing data of the sample to be tested.

As used herein, the predetermined sample referred to a nucleic acid sample known to have no CNV, that was, a normal pregnant woman's nucleic acid sample.

As used herein, the fitting curve represented a curve obtained by linearly fitting the relationship between the theoretical value of the copy ratio and the fetal concentration.

## Example 1: Modification of PCA denoising step to reduce false positive CNV reporting

In the experiment, through the library preparation and sequencing of the maternal source CNV abnormal sample, the sequencing data was improved by PCA denoising step. Finally, it was found that the improved PCA denoising step significantly reduced the false positive CNV reporting.

1) Sample collection: Some samples with maternal abnormalities and false positive CNV reports that appeared in the company's previous clinical test samples were selected.

2) Plasma separation: The peripheral blood collected in the first step was transferred to EDTA tube or cell-free DNA storage tube, the blood collection tube was centrifuged by a low-speed centrifuge at 1600g for 10 minutes, and the supernatant plasma was pipetted and transferred to EP tube; then the EP tube was centrifuged by a high-speed centrifuge at 16000g for 10 minutes, and the supernatant plasma was pipetted and transferred to a new EP tube, and temporarily stored in a -80 °C refrigerator for cell-free DNA extraction later.

3) Plasma cell-free DNA extraction: "Nucleic Acid Extraction Reagent" (BGI Biotechnology (Wuhan) Co., Ltd.) was used to extract plasma cell-free DNA. The plasma in step 2 was taken from the refrigerator, melt at room temperature, mixed well, and centrifuged briefly. 200 $\mu$L was taken and transferred into EP tube, added with 10 $\mu$L of proteinase K, 14 $\mu$L of magnetic beads and 280 $\mu$L of lysis solution, mixed well, and incubated at room temperature for 15 minutes. The magnetic beads were absorbed on a magnetic grate, and the waste liquid was discarded. Cleaning Solution 1 and Cleaning Solution 2 were added to wash the magnetic beads, 32 $\mu$L of elution buffer was added to elute nucleic acid, the magnetic beads were absorbed on a magnetic grate, and the DNA solution was pipetted and transferred to an EP tube.

39 μL of purified magnetic beads was added to EP tube, mixed well and allowed to stand for 5 minutes, and absorbed on a magnetic grate, the supernatant was taken and added to a new EP tube, 30 μL of purified magnetic beads were added, mixed well and allowed to stand for 5 minutes, and absorbed on a magnetic grate, and the supernatant was discarded. The magnetic beads were washed twice with 75% ethanol, added with 42 μL of elution buffer to elute nucleic acid, and the magnetic beads was absorbed on a magnetic graft. 40 μL of DNA solution was taken and transferred into a PCR tube, and used to perform the next step of library preparation.

4) Library preparation: The extracted DNA was subjected to end repair, adapter ligation, and PCR amplification for library preparation.

① End repair: 9.4 μL of end repair reaction solution and 0.6 μL of end repair enzyme were added to PCR tube, mixed well, centrifuged instantly, placed on a PCR instrument, incubated at 37°C for 10 min, incubated at 65°C for 15 min, cooled to 4°C and maintained. After the reaction was over, the PCR tube was taken out and centrifuged instantly.

② Adapter ligation: 24 μL of ligation reaction solution, 1 μL of ligase and 5 μL of label adapter were added to the PCR tube, mixed thoroughly and centrifuged instantly, placed on the PCR instrument and incubated at 23°C for 20 min, cooled to 4°C and maintained; 40 μL of purified magnetic beads was added for purification, 23 μL of elution buffer was added to elute the nucleic acid, the magnetic beads were absorbed on a magnetic grate, and 21 μL of DNA solution was taken and transferred into the PCR tube.

③ PCR amplification: 25 μL of PCR reaction solution and 4 μL of primers were added to the PCR tube, mixed thoroughly, centrifuged instantly, and placed on the PCR instrument to run the following program: 98°C, 2 minutes → (98°C, 15 seconds → 56°C, 15 seconds → 72°C, 30 seconds) 17 cycles → 72°C, 5 minutes → 4°C, infinity. 50 μL of purified magnetic beads were added for purification, and the concentration of the library sample was measured. The obtained library sample was temporarily stored in a 4°C refrigerator.

5) Sequencing reaction

After mixing equal amounts of library samples, MGISEQ-2000 sequencer or BGISEQ-500 sequencer manufactured by MGI was used for sequencing.

After obtaining the sequencing results, the downloaded data were subjected to data preprocessing, PCA denoising, HMM model detection for CNV, and CNV copy ratio calculation, and finally, the source of CNV abnormality was determined by comparing the theoretical value of copy ratio with the threshold.

6) Downloaded data: The downloaded data referred to the fastq data generated by the sequencer in the above experimental process.

7) Data preprocessing: Data preprocessing comprised the common operations of the conventional NIPT (non-invasive prenatal DNA testing) process. Specifically, it comprised the following steps:

① Sequence alignment and filtration: The sequence information contained in the fastq format file output by the sequencer was aligned to the human reference genome to filter out sequences with poor alignment quality and multiple alignment sequences, and leave the unique alignment sequences, and the coordinates and other information of each unique alignment sequence were stored in a bam format file.

② Window division and data correction: The reference genome has been divided into windows, except several windows required for subsequent detection, the read counts aligned to each window was counted, and the original depth of each window was GC-corrected according to the GC content of the window to obtain the corrected window depth information.

③ Calculation of fetal concentration: The proportion of fetal DNA in the cfDNA (cell-free DNA) sample was determined. For male fetuses, since pregnant women did not contain Y chromosome fragments, the proportion of Y chromosome fragments aligned in cfDNA could be used for judgment. For female fetuses, since the window distribution of chromosome fragments in fetal cfDNA was not exactly the same as that of maternal DNA, a large sample size machine learning model built based on this difference was used to determine the fetal concentration.

8) PCA denoising: The previously commonly used denoising method used the sample to be tested for PCA, and then the first ten-dimensional principal components of PCA were removed to denoise the sample. However, since PCA was an unsupervised learning method, it could not be guaranteed that all the first ten-dimensional principal components learned are experimental or analytical noises. When the sample to be tested had a serious abnormality, such as an abnormal number of maternal source chromosomes, the PCA method would mistakenly learn the abnormal information of these windows, thereby introducing some errors when removing the first ten-dimensional principal components, resulting in abnormal detection of CNV in some other intervals.

Therefore, in this experiment, this PCA denoising step used a fixed reference set sample to perform PCA learning on the principal component features representing noises, and used this fixed PCA result to perform denoising on the sample to be tested. For the effectiveness of PCA learning, the fixed reference set sample should maintain a high degree of similarity with the sample to be tested in terms of experimental conditions, data volume and other factors. Different reference set PCA results should be used for samples with different experimental processes, sequencing platforms or data volumes.

9) HMM model detection of CNV: The public XHMM software was used to detect CNV on the data processed above. The process used the sequencing depth information after denoising and normalization to predict CNV based on the hidden Markov model. The process would output the coordinates, posterior probability, z value and other information of the detected CNV. The detected CNV would be filtered and annotated based on this information, so that the result with shorter fragment and low prediction reliability was removed by filtration, and the result of maternal source CNV was annotated at the same time.

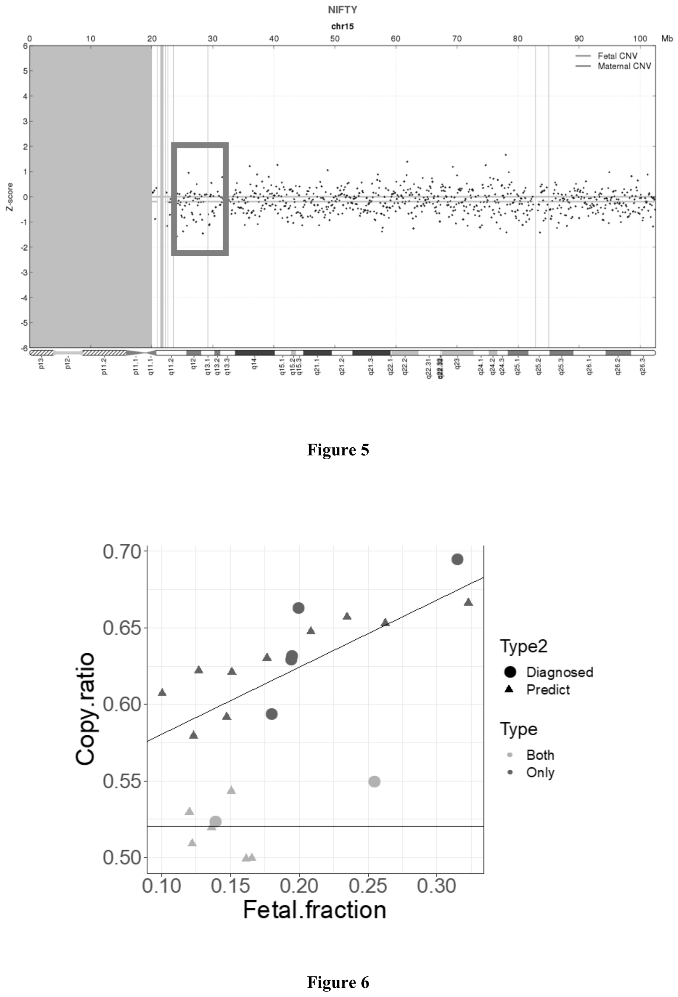

It was found in the results that in some samples with more maternal source X chromosomes, abnormal CNVs in the 15q11.2q12 and 16p11.1p11.2 intervals were detected, which was a common clinical problem before. Figure 4 showed the CNV detection results of chr15 of one of the samples, in which the framed part was abnormal interval, and it could be seen that in this interval, the process reported an abnormal deletion CNV. After processing with the new PCA denoising process, the copy-number of the sample to be tested in the previous false positive interval was within the normal standard (Figure 5), and no CNV positive was reported. That was, the new PCA denoising process could effectively solve this type of false positive problem of other autosomal CNVs caused by more abnormal maternal source X chromosomes.

**Example 2: Distinguishing maternal-fetal CNV and maternal CNV in maternal source DiGeorge sample**

First, the copy ratio (copy-number ratio) of each detected CNV was calculated for the data obtained in Example 1 to evaluate the difference between the copy-number of the CNV and the normal diploid region. When the CNV was a maternal source CNV, this indicator would be used to distinguish maternal CNV from maternal CNV later.

1) Calculation of copy ratio: Copy ratio was defined as a ratio of the copy-number of the target segment to the copy-number of the normal diploid interval. The calculation method was to divide the relative depth of the target segment of the sample to be tested after GC correction and depth correction by the relative depth of the corresponding segment of the normal sample in the fixed reference set. The formula was as follows:

Relative depth = mean read counts in the window of the CNV segment/mean read counts in all windows of the predetermined sample;

Copy ratio = relative depth of the CNV segment of the sequencing data of the predetermined sample/mean relative depth of the same segment of the sequencing data of the predetermined sample;

2) Determination of maternal-fetal CNV: Based on the copy ratio of the maternal source CNV and the fetal concentration, it was determined whether the CNV belonged to the maternal-fetal CNV or the maternal CNV. Theoretically, in the maternal-fetal CNV scenario where both the mother and the fetus carried CNV, the copy ratio of the CNV was irrelevant to the fetal concentration. For example, for deletion-type CNV, the copy ratio should be 0.5 regardless of the fetal concentration. In the scenario where the maternal CNV was carried only by the mother, the copy ratio of the CNV was closely related to the fetal concentration. For example, in the deletion-type CNV, the copy ratio of the CNV was proportional to the fetal concentration. The higher the fetal concentration, the larger the copy ratio, and the copy ratio should be higher than 0.5.

$$Copy\ ratio\ theoretical\ value = \begin{cases} 0.5 & maternal - fetal\ deletion\ CNV \\ 0.5 + f & maternal\ delection\ CNV \\ 1.5 - f & maternal\ duplication\ CNV \\ 1.5 & maternal - fetal\ duplication\ CNV \end{cases}$$

(f represents the fetal DNA concentration)

In order to verify this theory and determine the threshold, a total of 607 samples with maternal autosomal CNV deletions from our company's previous clinical test samples were selected to check the relationship between copy ratio and fetal concentration. The results showed that there were two types of samples, one showed that the copy ratio was proportional to the fetal concentration, which was consistent with the theory, and the other showed that the copy ratio had a low relationship with the fetal concentration, which was maintained at around 0.5. In samples with higher fetal concentrations, the difference between the two types of samples was more obvious. Therefore, according to the distribution of samples with fetal concentrations higher than 0.1, 0.55 was preliminarily determined as the threshold for maternal and fetal CNVs. Then, linear fitting was performed on the relationship between the copy ratio and fetal concentration of the preliminarily divided maternal and fetal CNVs, and the theoretical model of maternal and fetal CNVs was obtained. The formula used was as follows:

$$Deletion - type\ copy\ ratio\ theoretical\ model = \begin{cases} a + b * f & maternal\ CNV \\ c & maternal - fetal\ CNV \end{cases}$$

$$Duplication - type\ copy\ ratio\ theoretical\ model = \begin{cases} d + e * f & maternal\ CNV \\ j & maternal - fetal\ CNV \end{cases}$$

wherein, $f$ was the concentration of fetal cell-free DNA in the sample with maternal source CNV, and a, b, c, d, e, j were obtained by fitting the copy-number ratio of nucleic acid sample sequencing data with maternal CNV and the concentration of fetal cell-free DNA; wherein, b, e were the slopes of the fitting curves, a, d were the intercepts of the fitting curves, and c, j were constants.

For the sample to be tested, the fetal concentration of the sample was used to calculate the theoretical copy ratio of maternal and maternal-fetal CNVs respectively, and the threshold was set as the mean of the two theoretical values. The formula were as follows:

copy-number ratio threshold of deletion-type CNV = (theoretical value of copy-number ratio of maternal-fetal deletion-type CNV + theoretical value of copy-number ratio of maternal deletion- type CNV)/2;

copy-number ratio threshold of duplication-type CNV = (theoretical value of copy-number ratio of maternal-fetal duplication-type CNV + theoretical value of copy-number ratio of maternal duplication-type CNV)/2.

In the deletion-type CNV copy ratio theoretical model, when the copy ratio of the sample to be tested was higher than the threshold, it was an indication that the CNV came from the mother; when the theoretical value of the copy ratio of the sample to be tested was lower than the threshold, it was an indication that the CNV came from the mother and the fetus.

In the duplication-type CNV copy ratio theoretical model, when the copy ratio of the sample to be tested was higher than the threshold, it was an indication that the CNV came from the mother and the fetus; when the theoretical value of the copy ratio of the sample to be tested was lower than the threshold, it was an indication that the CNV came from the mother.

By using the samples carrying maternal DiGeorge detected by our company in actual clinical testing, sequencing was performed for a total of 23 times, and the copy ratios were calculated using the process of this experiment. The results were shown in Figure 6. The two curves in the figure were the theoretical models between maternal and maternal source CNV copy ratios and fetal concentrations. It could be seen that these samples had good discrimination, among which the copy ratio of sample judged to be maternal CNV had a good positive correlation with the fetal concentration, which also had a good degree of consistency with the theoretical value model.

In the description of the present application, the reference terms "one embodiment", "some embodiments", "example", "specific example", or "some examples" means that the specific features, structures, materials or characteristics described in conjunction with the embodiment or example are included in at least one embodiment or example of the present invention. In the description, the schematic representation of the above terms does not necessarily refer to the same embodiment or example. Moreover, the specific features, structures, materials or characteristics described can be combined in any one or more embodiments or examples in a suitable manner. In addition, in the absence of contradiction, a person skilled in the art can combine different embodiments or examples described in the description and the features of different embodiments or examples.

Although the embodiments of the present invention have been shown and described above, it should be understood that the above embodiments are exemplary and cannot be understood as limiting the present invention. Ordinary technicians in this field can change, modify, replace and modify the above embodiments within the scope of the present invention.

**Claims**

1. A method for detecting chromosome copy-number variant, **characterized by**, comprising:

   the sequencing data of a sample to be tested is subjected to PCA denoising processing and CNV analysis processing to detect whether the sample contains CNV and/or determine the source of CNV;
   wherein, the PCA denoising processing is performed by comparing the sequencing data of the sample to be tested with a reference data set, and the reference data set is the principal component feature representing the noises obtained after PCA learning of a predetermined sample.

2. The method according to claim 1, **characterized in that** the sample to be tested and the predetermined sample are both nucleic acid sample.

3. The method according to claim 2, **characterized in that** the nucleic acid sample comprises at least one selected from DNA and RNA.

4. The method according to claim 2, **characterized in that** the nucleic acid sample is derived from a peripheral blood of a pregnant woman.

5. The method according to claim 4, **characterized in that** the nucleic acid sample is cell-free DNA in a peripheral blood plasma of a pregnant woman.

6. The method according to claim 2, **characterized in that** the predetermined sample is a sample known not to have CNV.

7. The method according to claim 1, **characterized in that** the sequencing data of the sample to be tested and the reference data set of the predetermined sample are obtained under identical conditions or with the same data volume.

8. The method according to claim 1, **characterized in that** the CNV analysis processing comprises:

   performing CNV prediction processing on the sequencing data after PCA denoising processing;
   performing filtration processing on the CNV prediction processing result;
   performing copy-number ratio theoretical value and threshold analysis on the filtered CNV prediction processing result;
   determining the source of the CNV based on the copy-number ratio theoretical value and threshold analysis result.

9. The method according to claim 8, **characterized in that** the CNV prediction processing is performed using a hidden Markov model.

10. The method according to claim 8, **characterized in that** the CNV prediction processing result comprises the coordinates, posterior probability, and z value information of the CNV.

11. The method according to claim 8, **characterized in that** the filtration processing comprises: removing short fragment and low-reliability CNV prediction processing result based on the coordinates, posterior probability, and z value information of the CNV.

12. The method according to claim 1, **characterized in that** the chromosome copy-number variant comprises DNA fragment deletion and DNA fragment duplication.

13. The method according to any one of claims 8 to 12, **characterized in that** when the sample to be tested is a sample with maternal CNV determined by the method according to any one of claims 8 to 12, the theoretical value of the copy-number ratio is calculated by the following formula:

$$\text{Deletion} - \text{type copy ratio theoretical model} = \begin{cases} a + b * f & \text{maternal CNV} \\ c & \text{maternal} - \text{fetal CNV} \end{cases}$$

$$\text{Duplication} - \text{type copy ratio theoretical model} = \begin{cases} d + e * f & \text{maternal CNV} \\ j & \text{maternal} - \text{fetal CNV} \end{cases}$$

wherein, $f$ is the concentration of fetal cell-free DNA in a sample with maternal source CNV, and a, b, c, d, e, j are obtained by fitting the copy-number ratio of nucleic acid sample sequencing data with maternal CNV and the concentration of fetal cell-free DNA;
wherein, b, e are the slopes of the fitting curves, a, d are the intercepts of the fitting curves, and c, j are constants;
wherein, the copy-number ratio = relative depth of the CNV segment of the sequencing data of the predetermined sample / mean relative depth of the same segment of the sequencing data of the predetermined sample;
the relative depth is calculated by the following formula:

Relative depth = mean read counts in the window within the CNV segment/mean read counts in all windows of the predetermined sample.

14. The method according to claim 13, **characterized in that** the copy-number ratio threshold is calculated by the following formula:

copy-number ratio threshold of deletion-type CNV = (theoretical value of copy-number ratio of maternal-fetal deletion-type CNV + theoretical value of copy-number ratio of maternal deletion-type CNV)/2;

copy-number ratio threshold of duplication-type CNV = (theoretical value of copy-number ratio of maternal-fetal duplication-type CNV + theoretical value of copy-number ratio of maternal duplication-type CNV)/2.

15. The method according to claim 14, **characterized in that**, determining the source of the CNV based on the comparison result between the theoretical value of the copy-number ratio and the threshold comprises:

(1) in a deletion-type CNV, when the theoretical value of the copy-number ratio is higher than the threshold, it is an indication that the CNV comes from the mother;
(2) in a deletion-type CNV, when the theoretical value of the copy-number ratio is lower than the threshold, it is an indication that the CNV comes from the mother and the fetus;
(3) in a duplication-type CNV, when the theoretical value of the copy-number ratio is higher than the threshold, it is an indication that the CNV comes from the mother and the fetus;
(4) in a duplication-type CNV, when the theoretical value of the copy-number ratio is lower than the threshold, it is an indication that the CNV comes from the mother.

16. The method according to claim 1, **characterized in that** the sequencing data of the sample to be tested is obtained in the following manner:

constructing a sequencing library based on the sample to be tested;
performing sequencing processing on the sequencing library to obtain the sequencing data of the sample to be tested.

17. The method according to claim 16, **characterized in that** the sequencing processing is performed on MGISEQ-2000 sequencer or BGISEQ-500 sequencer as manufactured by MGI.

18. The method according to claim 16, **characterized in that** the construction of the sequencing library comprises performing end repair processing, adapter ligation processing and PCR amplification processing on the sample to be tested.

19. The method according to claim 18, **characterized in that** the end repair processing is performed in the presence of an end repair enzyme.

20. The method according to claim 18, **characterized in that** the adapter ligation comprises ligating the sample to be tested that has undergone end repair with the label adapter in the presence of a ligase.

21. The method according to claim 18, **characterized in that** the PCR amplification comprises performing a PCR reaction on the sample to be tested that is ligated with the label adapter.

22. The method according to claim 21, **characterized in that** the PCR reaction conditions comprise: 98°C, 2 minutes → (98°C, 15 seconds → 56°C, 15 seconds → 72°C, 30 seconds) 17 cycles → 72°C, 5 minutes → 4°C.

23. A system for detecting chromosome copy-number variant, **characterized by**, comprising:

    a PCA denoising device, in which the PCA denoising device is used to perform PCA denoising on the sequencing data of the sample to be tested;
    a CNV analysis device, in which the CNV analysis device is connected to the PCA denoising device, and is used to perform CNV analysis on the sequencing data after PCA denoising, so as to determine whether there is CNV and/or CNV source;
    wherein, the PCA denoising is performed by comparing the sequencing data of the sample to be tested with a reference data set, and the reference data set is the principal component feature representing the noise obtained after PCA learning of the predetermined sample.

24. The system according to claim 23, **characterized in that** the CNV analysis device comprises:

    a CNV prediction processing unit, in which the CNV prediction processing unit is used to perform CNV prediction processing on the sequencing data after PCA denoising processing;
    a filtration unit, in which the filtering unit is connected to the CNV prediction processing unit, and is used to filter the CNV prediction processing result;
    a copy-number ratio analysis unit, in which the copy-number ratio analysis unit is connected to the filtering unit, and is used to perform copy-number ratio theoretical value and threshold analysis on the CNV prediction processing result after filtration processing;
    a judgment unit, in which the judgment unit is connected to the copy-number ratio analysis unit, and is used to determine the source of the CNV based on the copy-number ratio theoretical value and threshold analysis results.

25. The system according to claim 23, **characterized by**, further comprising a sequencing library construction device and a sequencing device, wherein the sequencing library construction device is used to construct a sequencing library based on the sample to be tested; the sequencing device is connected to the sequencing library construction device, and the sequencing device is connected to the PCA denoising device, and is used to sequence the sequencing library to obtain the sequencing data of the sample to be tested.

26. An electronic device for detecting chromosome copy-number variant, **characterized by**, comprising a memory and a processor; wherein the processor runs a program corresponding to an executable program code by reading the executable program code stored in the memory, so as to implement the method for detecting chromosome copy-number variant as claimed in claim 1.

27. A computer-readable storage medium, wherein the computer-readable storage medium stores a computer program, **characterized in that** the program implements the method for detecting chromosome copy-number variant as claimed in claim 1 when executed by the processor.

PCA denoising device — 100

CNV analysis device — 200

**Figure 1**

CNV prediction processing unit — 201

Filtration unit — 202

Copy-number ratio analysis unit — 203

Judgment unit — 204

**Figure 2**

**Figure 3**

**Figure 4**

**Figure 5**

**Figure 6**

## INTERNATIONAL SEARCH REPORT

| International application No. |
|---|
| **PCT/CN2023/123292** |

### A. CLASSIFICATION OF SUBJECT MATTER

G16B 40/00(2019.01)i

According to International Patent Classification (IPC) or to both national classification and IPC

### B. FIELDS SEARCHED

Minimum documentation searched (classification system followed by classification symbols)

IPC: G16B

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: pca, 主成分分析, cnv, 拷贝数变异, 测序, 参照, 参考, 噪声, 降噪, 染色体, 核酸, 样本, 待测, chromosome, copy number variation, detect, noise, reduction, sequence, sample, reference, principal component

### C. DOCUMENTS CONSIDERED TO BE RELEVANT

| Category* | Citation of document, with indication, where appropriate, of the relevant passages | Relevant to claim No. |
|---|---|---|
| PX | CN 116013419 A (BGI GENOMICS CO., LTD. et al.) 25 April 2023 (2023-04-25) claims 1-15 | 1-27 |
| X | CN 112669901 A (BEIJING USCI MEDICAL LABORATORY CO., LTD.) 16 April 2021 (2021-04-16) description, paragraphs [0004]-[0061] and [0071]-[0162] | 1-27 |
| A | CN 113674803 A (GUANGZHOU BURNING ROCK MEDICAL EXAMINATION INSTITUTE CO., LTD.) 19 November 2021 (2021-11-19) entire document | 1-27 |
| A | CN 111210873 A (XI'AN JIAOTONG UNIVERSITY) 29 May 2020 (2020-05-29) entire document | 1-27 |
| A | US 2019371428 A1 (SEQUENOM, INC.) 05 December 2019 (2019-12-05) entire document | 1-27 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 December 2023** | **21 December 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2023/123292**

| Patent document cited in search report | | | Publication date (day/month/year) | Patent family member(s) | | | Publication date (day/month/year) |
|---|---|---|---|---|---|---|---|
| CN | 116013419 | A | 25 April 2023 | None | | | |
| CN | 112669901 | A | 16 April 2021 | None | | | |
| CN | 113674803 | A | 19 November 2021 | HK | 40057523 | A0 | 14 April 2022 |
| | | | | WO | 2023030233 | A1 | 09 March 2023 |
| CN | 111210873 | A | 29 May 2020 | None | | | |
| US | 2019371428 | A1 | 05 December 2019 | WO | 2018136882 | A1 | 26 July 2018 |
| | | | | CA | 3049457 | A1 | 26 July 2018 |
| | | | | EP | 3571614 | A1 | 27 November 2019 |
| | | | | CA | 3194621 | A1 | 26 July 2018 |
| | | | | HK | 40012484 | A0 | 24 July 2020 |

Form PCT/ISA/210 (patent family annex) (July 2022)